# EUROPÄISCHE PATENTSCHRIFT

(11) **EP 1 163 004 B1**
(45) Veröffentlichungstag und Bekanntmachung des Hinweises auf die Patenterteilung: **05.03.2003**
(21) Anmeldenummer: 00929248.3
(22) Anmeldetag: 23.03.2000
(51) Int. Cl.: A61K 39/44, B01J 20/22, A61M 1/36, C07K 1/22, C07K 16/02, C07K 16/18, C07K 16/24

(54) **IMMUNADSORBER ZUR SEPSISTHERAPIE**
IMMUNOADSORBER FOR USE IN SEPSIS THERAPY
IMMUNO-ADSORBANTS POUR LE TRAITEMENT DE LA SEPTICEMIE

(30) Priorität: 26.03.1999 DE 19913707
(43) Veröffentlichungstag der Anmeldung: 19.12.2001
(73) Patentinhaber: Bioserv AG, 18059 Rostock (DE)
(72) Erfinder: HEINRICH, Hans-Werner, D-17498 Riemserort (DE); HAHN, Hans-Jürgen, D-17495 Karlsburg (DE); MEYER, Udo, D-18239 Hastorf (DE); KRUSCHKE, Peter, D-17498 Insel Riems (DE); WAGNER, Heinz-Jürgen, D-13125 Berlin (DE)
(74) Vertreter: Baumbach, Friedrich, Dr.
(86) Internationale Anmeldenummer: DE0000927
(87) Internationale Veröffentlichungsnummer: WO00058005

(56) Entgegenhaltungen:
- EP-A- 0 300 740
- WO-A-94/21124
- GB-A- 2 251 187
- US-A- 5 679 775
- HOFFMANN J.N. ET AL: "Effect of hemofiltration on hemodynamics and systemic concentrations of anaphylatoxins and cytokines in human sepsis." INTENSIVE CARE MEDICINE, (1996) 22/12 (1360-1367). , XP000939172

## Beschreibung

Die Erfindung betrifft Immunadsorber zur Sepsistherapie, insbesondere zur Entfernung von Komplementfaktoren und Lipopolysacchariden (LPS) sowie ggf. von TNF und Interleukinen aus Körperflüssigkeiten, Verfahren zu ihrer Herstellung und ihre Verwendung.

Jährlich erkranken in den USA, Japan und der EU ca. 3.5 Mill. Patienten an Sepsis. Bei einer Gesamteinwohnerzahl von 785 Mill. liegt die Inzidenz für diese Länder unter 0,5%. Wenn jedoch hospitalisierte Patienten hinsichtlich der Erkrankungshäufigkeit untersucht werden, findet man 2,0 ± 0,16 Sepsisfälle pro 100 Krankenhausaufnahmen. Die enorme gesundheitspolitische und individuelle Bedeutung ergibt sich aus der Beobachtung, daß ca. 25 % dieser Patienten auch bei intensivster medizinischer Betreuung durch hochqualifizierte Spezialisten in modern ausgerüsteten Einrichtungen (Intensivstationen) das Syndrom des septischen Schocks erleiden, das durch eine Letalitätsrate von >45% charakterisiert ist.

Insbesondere bei polytraumatisierten Patienten (Verkehrsunfälle, Verbrennung, schwere Operationen) ist das Erkrankungsrisiko für den septischen Schock sehr hoch. Neben der Infektion von außen ist das Durchbrechen der Darmbarriere für normalerweise im Darm vorkommende gram-negative Bakterien infolge eines partiellen Funktionsverlustes des Immunsystems dieser Patienten und somit eine Infektion von "innen" nachzuweisen.

In mehr als 50% der Erkrankungen lösen gram-negative Bakterien bzw. deren Zellwandbestandteile, die Endotoxine (Lipopolysaccharide, LPS), den septischen Schock aus. Das von den Bakterien freigesetzte LPS bindet sich an ein Serumprotein (LBP), um danach von den LPS-Rezeptoren der Monocyten/Makrophagen (CD14) aufgenommen zu werden. Die so aktivierten CD14+ Zellen produzieren Zytokine (TNFα, interleukin-1 (IL-1), IL-6, IL-8). die via Zytokin-Rezeptor der Zielzelle ihre Wirkung ausüben.

Parallel zur Stimulierung der Monozyten und Makrophagen wird das Komplementsystem aktiviert. Es ist ein integrierter Bestandteil der immunologischen Abwehr der Säugetiere zur unmittelbaren und unspezifischen Bekämpfung bakterieller Mikroorganismen und Fremdpartikel. Von den im Blutserum vorkommenden Komplementproteinen, vorrangig Proenzyme, die durch proteolytische Spaltung aktiviert werden, spielt das C3-Protein mit einer Serumkonzentration von ca. 1g/l eine zentrale Rolle. Nach Kontakt der Mikroorganismen mit dem C3 wird das Komplementprotein C3a abgespalten und durch das entstehende C3b wird einerseits die Bildung der C5-Konvertase eingeleitet (alternativer Weg der Komplementaktivierung) und andererseits die Reaktion dadurch amplifiziert, indem das C3b durch Anlagerung von Serumfaktoren sich zur C3-Konvertase wandelt. Das ebenfalls im Serum vorkommende Komplementprotein C5 wird durch die nun verstärkt bereitgestellte C5-Konvertase proteolytisch unter Bildung von C5a gespalten. An das entstandene C5b lagern sich weitere Komplementproteine (C6-C9) an, bis schlußendlich der polymere hydrophobe Membranangriffskomplex (MAK) gebildet wurde, der sich in die Bakterienmenbran (Opsonidierung) einlagert und Poren bildet, die zur Phagozytose und damit zur Elimination der Mikroorganismen (und des gebundenen MAK) führen. Die im Prozeß der Komplementaktivierung freigesetzten Komplementfaktoren C3a und C5a (Anaphylatoxine) bewirken durch eine Erhöhung der Gefäßpermeabilität und die durch sie induzierte Freisetzung von Chemotoxinen das Anlokken der phagozytierenden Zellen an den Ort des bakteriellen Befalls. Die Verringerung der Anzahl an Bakterien bewirkt die Verminderung der Aktivierung des Komplementsystems. Diese unmittelbare und unspezifische Reaktion ist mit den anderen immunologischen Abwehrsystemen insofern eng verwoben, als daß beispielsweise durch Komplementfaktoren die Synthese und Freisetzung der für die zelluläre Abwehr essentiellen Zytokine reguliert wird. Um die inflammatorische Wirkung zu vermitteln, werden C3a und C5a an spezifische zellständige Rezeptoren gebunden, die wiederum in Abhängigkeit von der Immunreaktivität unterschiedlich stark exprimiert werden. Um die Immunabwehr permanent reaktionsbereit zu erhalten, sind aktivierte Komplementfaktoren nicht nur nach Befall mit Mikroorganismen nachweisbar, sondern integrierter Bestandteil des Serums von Normalpersonen in einer Konzentration von 1 - 10 ng/ml.

Insbesondere bei einer entwickelten Sepsis, bei akutem Lungenversagen und bei moribunden Patienten können die Plasmaspiegel der Anaphylatoxine mehr als tausendfach erhöht sein.

Fast ausschließlich auf der Basis von in vitro-Untersuchungen existieren unterschiedlichste, meistens unspezifisch wirkende Lösungsvarianten, um die Wirkungen verschiedener Komplementfaktoren zu eliminieren, die aber wegen der zu erwartenden Nebenwirkungen kaum unter in vivo Bedingungen getestet werden können (z.B. WO-A-98/34959).

In ex vivo Verfahren zur Prävention der Komplementaktivierung durch künstliche, extrakorporale Oberflächen (z.B. Oberflächenbeschichtungen) wurde eine unspezifische Komplementinaktivierung erfolgreich durchgeführt. Des weiteren ist aus US 5,853,722 die selektive Entfernung aktivierter Komplementfaktoren unter Nutzung von spezifischen C5 Antikörpern bekannt und sicher auch zu bevorzugen, zumal hochaffine Antikörper zwischenzeitlich gegen alle Komponenten des Komplementsystems generiert wurden.

Die aufgezeigte funktionelle Kaskade dient vornehmlich der Eliminierung der in den Organismus eingedrungenen Bakterien. Sobald jedoch eine Diskrepanz zwischen der Anzahl und/oder Virulenz der eingedrungenen Bakterien und der Eliminierungskapazität des Immunsystems (z.B. beim posttraumatischen Immundefizit) auftritt, wird eine überschießende Aktivierung beobachtet, die nachfolgend von einer massenhaften Freisetzung von "Schockmediatoren" (Interleukine, Thrombozytenaktivierunsfaktor (PAF), aber auch Sauerstoffradikale, Prostaglandine und deren Stoffwechselprodukte) begleitet wird, die die Eliminierungskapazität für LPS weiter einschränkt. Zusätzlich werden durch das LPS auch CD14-negative Zellen (z.B. Endothelien) aktiviert, da im Blutplasma lösliches CD14 (sCD14) als LPS Fänger vorhanden ist, das die Bindung an diese Zellen erleichtert und die Bildung und Freisetzung weiterer Schockmediatoren induziert, die dadurch den Circulus vitiosus verstärken. Da die Schockmediatoren zwar selektiv, aber nicht spezifisch wirken, werden Funktionseinschränkungen in verschiedenen Zellen und Organen beobachtet (Blutgerinnungsystem, Kreislauf, Komplement-System), so daß die den gesamten Organismus befallenen Entzündungsreaktionen die Schockgenese einleiten, die zu irreversiblen Organschäden, zum Kreislaufzusammenbruch und zum Tod führen.

Um diese Funktionskette zu durchbrechen sind unterschiedliche Therapiestrategien untersucht worden.
Die Unterbrechung der Kaskade mit Antikörpern, die die LPS-Bindung an Proteine (LBP, sCD14), an den Rezeptor (CD14), an freigesetzte Zytokine oder an Zytokinrezeptoren unterbrechen bzw. mit Antagonisten, welche die funktionellen Bereiche der Rezeptoren blockieren, erbrachten an verschiedenen tierexperimentellen Sepsismodellen zwar beeindruckende Erfolge, jedoch bis heute liegen keine klinisch erprobten, erfolgreichen Präventions- und/oder Therapiestudien vor.

Die hochgesteckten Erwartungen konnten nicht erfüllt werden, da zunehmend erkannt werden mußte, daß LPS auch Zellen und Gewebe beeinflußt und in ihrem Funktionszustand verändert, die durch diese Therapieansätze nicht beeinträchtigt werden. Außerdem muß berücksichtigt werden, daß ein durch einen Antikörper/Antagonisten inaktiviertes LPS (Immunkomplex) eliminiert werden muß, um eine biologische Reaktivität permanent auszuschließen. Die Eliminierung ist aber auch eine Funktion des Immunsystems, das, da stark geschwächt, dieser Aufgabe kaum oder nur sehr unvollkommen nachkommen kann.

Die Entwicklung des septischen Schocks ist ein sehr dynamisches Geschehen primär unterschiedlicher Genese, bei dem innerhalb kurzer Zeit unterschiedliche Mediatoren sehr verschiedene Reaktionen bewirken, die nach anfänglich lebenserhaltender Funktion rasch durch Fehlregulation zur Ausprägung des septischen Schocks führen.

Im Dokument EP-A-0 300 740 ist ein Immunadsorber zur Entfernung von Komplementfaktor C5a zur Blutreinigung beschrieben, bei dem monoklonale Antikörper an Polystyrol eingesetzt werden.

Aus US-A- 5 679 775 ist bekannt LPS aus Blut zur Sepsistherapie mittels Kationen- oder Anionenaustauschern zu entfernen, von der Verwendung von LPS spezifischen Antikörpern wird abgeraten.

Der Erfindung lag deshalb die Aufgabe zugrunde, ein modular aufgebautes Immunadsorptionssystem insbesondere für die extrakorporale Detoxifikation zu entwikkeln, das es ermöglicht, patientenspezifisch Plasma- und Gewebespiegel zu reduzieren.

Die Erfindung beruht unter anderem auf der Erkenntnis, daß TNFα eine Schlüsselstellung in diesem Regulationssystem einnimmt. Es wird durch unterschiedlichste "äußere" Einflüsse, wie z.B. Verletzungen, Entzündungen, Infekttonen, Septikämie u.a. von Makrophagen freigesetzt und induziert über eine Zytokinkaskade (IL-1, IL-6) eine lokale und systemische Aktivierung des unspezifischen und spezifischen Abwehrsystems. Klinisch äußert sich eine massive TNFα-Freisetzung in erhöhter Körpertemperatur, Inappetenz und allen Folgesymptomen einer katabolischen Stoffwechsellage. In der Pathogenese der Sepsis scheint in der frühen Phase dieser Erkrankung die Aktivierung der Makrophagen und damit die TNFα-Freisetzung für das Überleben des Patienten von essentieller Bedeutung zu sein, während im weiteren Verlauf der anhaltende Aktivierungszustand die Dekompensation aller Abwehrreaktionen nach sich zieht.

Die Aufgabe der Erfindung wurde durch einen Immunadsorber zur Sepsistherapie gelöst. Insbesondere dient der erfindungsgemäße Immunadsorber zur Entfernung von Komplementfaktoren und Lipopolysacchariden (LPS) sowie ggf. zur Entfernung von weiteren Sepsis-Mediatoren, wie von TNF und Interleukinen aus Körperflüssigkeiten. Er ist gekennzeichnet durch Trägermaterialien aus organischen oder synthetischen Polymeren, an die sowohl poly- oder monoklonale Antikörper gebunden sind, die gegen die Komplementfaktoren C3a und/oder C5a gerichtet sind, als auch Antikörper, die gegen Lipopolysaccharide (LPS) gerichtet sind. In einer bevorzugten Ausführung sind auch Antikörper, die gegen weitere Sepsis-Mediatoren gerichtet sind, an die Träger gebunden.

Bevorzugt handelt es sich um polyklonale Antikörper besonders bevorzugt um aviäre Antikörper des Typs IgY. Die Antikörper gegen Sepsis-Mediatoren sind entsprechend des Zustandes der Dysregulation enthalten.

Gemäß der Erfindung handelt es sich dabei um Antikörper, die gegen TNF, IL1, IL6, IL8 und/oder IL10 gerichtet sind.

Bevorzugte Antikörper gegen den Komplementfaktor C3a weisen spezifische Aktivität gegen mindestens eine der folgenden Peptidsequenzen auf:

NH2-KCCEDGMRQNPMR-COOH
NH2-RFSCQRRTRFISL-COOH
NH2-ITELRRQHARAS-COOH

Bevorzugte Antikörper gegen den Komplementfaktor C5a besitzen spezifische Aktivität gegen mindestens eine der folgenden Peptidsequenzen:

NH2-QADYKDDDDKLPAE-COOH
NH2-DDKLPAEGLDIENS-COOH

Bevorzugte Antikörper gegen IL1 α/β besitzen spezifische Aktivität gegen mindestens eine der folgende Peptidsequenzen

NH2-NCYSENEEDSSSID-COOH
NH2-GAYKSSKDDAKIT-COOH
NH2-WETHGTKNYFTS-COOH
NH2-RISDHHYSKGFRQA-COOH
NH2-VQGEESNDKIPVA-COOH
NH2-ESVDPKNYPKKKMEKRF-COOH

Bevorzugte Antikörper gegen IL6 besitzen spezifische Aktivität gegen mindestens eine der folgende Peptidsequenzen

NH2-APHRQPLTSSERIDKQI- COOH
NH2-QNRFESSEEQARA- COOH
NH2-AITTPDPTTNAS- COOH

Bevorzugte Antikörper gegen IL10 besitzen spezifische Aktivität gegen mindestens eine der folgende Peptidsequenzen

NH2-SPGQGTQSENSCT-COOH
NH2-QMKDQLDNLLLKES-CCOH
NH2-MPQAENQDPDIKA-COOH
NH2-LPCENKSKAVEQ-COOH

Bevorzugte Antikörper gegen TNFα besitzen spezifische Aktivität gegen mindestens eine der folgende Peptidsequenzen

NH2-VRSSSRTPSDKPVA-COOH
NH2-KSPCQRETPEGAEAKPW-COOH

Der erfindungsgemäße Immunadsorber weist als Trägermaterialien an sich übliche Membranen oder Partikel aus organischen oder synthetischen Polymeren auf, so z.B. aus Polystyrolen, Kohlenhydraten, wie z.B. Zellulose- oder Agarosederivate, oder aus Acrylaten, wobei die spezifischen Antikörper kovalent an diese gebunden oder über Spacer oder Linker an sie fixiert sind.

Die Herstellung der erfindungsgemäßen Immunadsorber erfolgt durch an sich bekannte Methoden, indem die Antikörper, die gegen C3a und/oder C5a und LPS sowie ggf. gegen weitere Sepsis-Mediatoren gerichtet sind, kovalent oder adsorptiv an die Trägermaterialen aus organischen oder synthetischen Polymeren gekoppelt werden.

Die spezifischen Antikörper werden durch an sich bekannte Immunisierung vorzugsweise von Kleinsäugern, wie Mäusen, Ratten oder Kaninchen oder Vögeln, wie z.B. Hühnern, mit den entsprechenden Antigenen hergestellt.

Gegenstand der Erfindung ist auch die Verwendung der Immunadsorber in Vorrichtungen zur Entfernung von Komplementfaktoren, LPS und ggf. von weiteren Mediatoren aus Körperflüssigkeiten, wie Blutplasma, in Abhängigkeit der patientenspezifischen Situation.

Bevorzugt werden die lmmunadsorber in der Sepsistherapie für die Plasmapherese bei Patienten mit Sepsis oder septischem Schock eingesetzt.

Obwohl für die meisten Substanzen Antikörper verfügbar sind, die nach bekannten Methoden an die unterschiedlichen Träger gekoppelt werden können, werden bevorzugt aviäre Antikörper verwendet, da diese im Gegensatz zu Säuger-Antikörpern das Komplementsystem nicht aktivieren. Da die aktivierenden Eigenschaften an den F_{c}-Teil der Säuger-Antikörper gebunden sind, kann prinzipiell auch das mit Papain abgespaltene F_{ab}-Fragment verwendet werden.

Nach derzeitigem Kenntnisstand haben immobilisierte aviäre Antikörper keinerlei unspezifische Wirkungen auf das Abwehrsystem des Menschen. Vögel, vorzugsweise Hühner werden mit üblichen Verfahren ohne oder mit Verwendung von Adjuvantien immunisiert. Die spezifischen Immunglobuline werden im Eidotter ausgeschieden und können hieraus mit üblichen Methoden isoliert werden. Sie werden mit bekannten Verfahren kovalent über den Fc-Teil an Mikropartikel oder Membranen gebunden.

Mit dem erfindungsgemäßen Immunadsorptionssystem für die extrakorporale Detoxifikation steht erstmals ein selektives System zur Verfügung, welches patientenspezifisch einsetzbar ist und durch das Fehlregulationen des Immunsystems behoben werden können.

Die Erfindung wird durch folgende Beispiele näher erläutert:

### Beispiel 1

### Herstellung polyklonaler Antikörper mittels immunogener Peptide:

Mittels einer Festphasensynthese werden die in Tab. 1 aufgelisteten Peptide hergestellt:

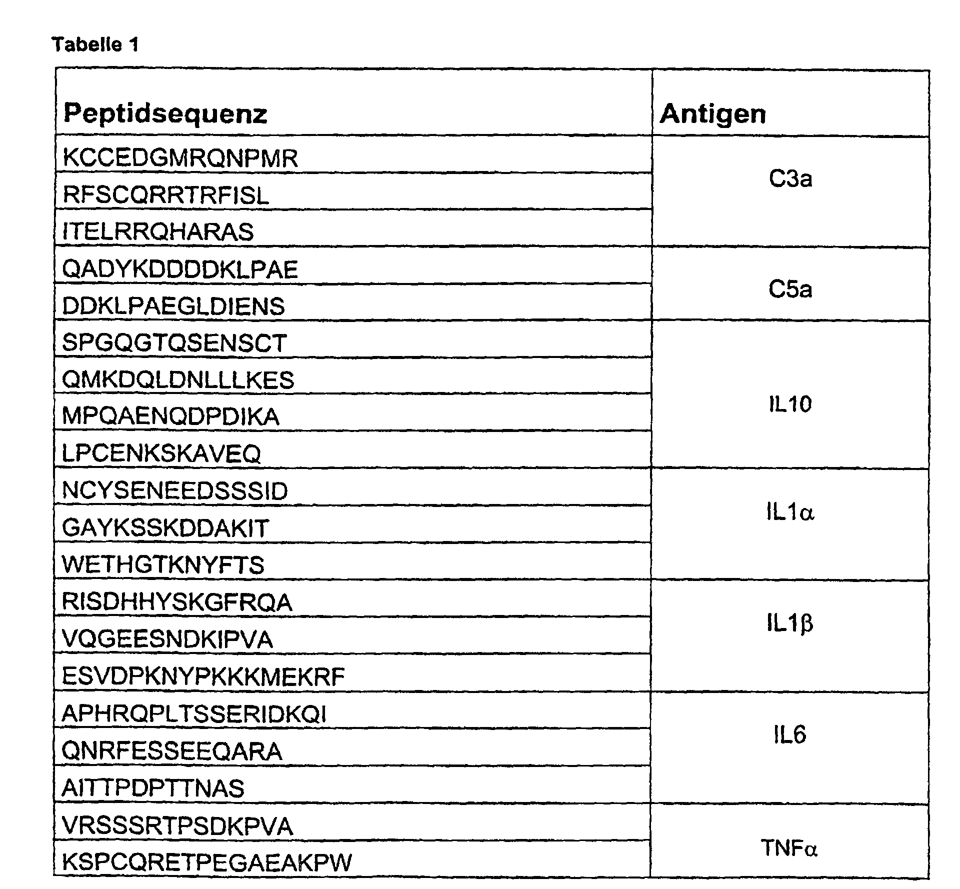

Diese Peptide werden nach Standardrezeptur kovalent an einen Träger (KLH) gebunden. Das Konjugat wird in PBS gelöst zu gleichen Teilen mit Freund's-Adjuvans gemischt. Die einzelne Impfdosis wird so eingestellt, dass sie jeweils 200µg des zum entsprechenden Antigen gehörenden Peptids enthält. Mit diesen Gemischen werden 15 Wochen alte Junghennen s.c. immunisiert und im Abstand von 4 Wochen 4 mal geboostert.

### Beispiel 2

### Herstellung polyklonaler Antikörper mittels Lipopolysacchariden (LPS)

Gereinigte LPS (SIGMA) von E. coli J5 werden in PBS gelöst und zu gleichen Teilen mit Freund's-Adjuvans gemischt. Mit diesem Gemisch werden 15 Wochen alte Junghennen s.c. immunisiert. Die LPS-Dosis beträgt pro Immunisierung 1 mg LPS. Im Abstand von 4 Wochen wird 4 mal geboostert.

### Beispiel 3

### Gewinnung der Antikörper (IgY) aus Eidotter:

Die Eier aus den Gelegen der immunisierten Hennen werden gesammelt. Nach Separation der antikörperhaltigen Eidotter erfolgt die Lagerung bei -20°C. Entsprechend dem Bedarf werden die Dotter aufgetaut und nach folgendem Schema aufbereitet (C.SCHWARZKOPF, B.THIELE (1996) ALTEX 13 Suppl. 16, 35-3):

| | | |
|---|---|---|
| A | TBS: 20 mM Tris/HCl, pH 7,5, 0,5 M NaCl | |
| B | 10 % (w/v) Dextransulfat in A | *Lösungen* |
| C | 1 M CaCl₂ | |
| D | 0,5 M EDTA, pH 7,5 | |
| E | gesättigte Ammoniumsulfat-Lösung | |

Das Eigelb (entspricht einem Volumen von 10 - 20 ml/Eigelb) wird in 100 ml TBS je Eigelb suspendiert. Lipide und Lipoproteine werden mit Dextransulfat (6 ml B je 100 ml TBS/Eigelb-Suspension) und Ca⁺⁺ (15 ml C je 100 ml TBS-Eigelb-Suspension) gefällt, 30 bis 60 Min. bei Raumtemperatur gerührt und mit 5.000 g abzentrifugiert. Das Pellet wird mit einem kleinen Volumen TBS (ca. 20 mlg/Eigelb) gewaschen und wieder zentrifugiert.

Die vereinigten Überstände werden durch ein Papierfilter filtriert, dann wird zum Filtrat 0,5 M EDTA bis zu einer Endkonzentration von ca. 30 mM EDTA (6 ml je 100 ml) gegeben, um restliche Ca⁺⁺-lonen zubinden. Anschließend wird der Überstand mit 24,3 g Ammoniumsulfat je 100 ml (entspricht 40% Sättigung) versetzt und 30 min. bei +4° C inkubiert. Der entstandene Niederschlag (IgY) wird zuerst mit 30% (NH₄)₂SO₄ (30 ml E + 70 ml dest. Wasser) gewaschen, zentrifugiert, dann im kleinstmöglichen Volumen TBS gelöst (ca. 10 ml / eingesetztes Eigelb) und gegen TBS dialysiert.
Der Gehalt an IgY wird photometrisch bei 275 nm bestimmt.

### Beispiel 4

### a) Aktivierung eines Trägers:

Die nach Beispiel 3 gereinigten IgY werden kovalent an einen geeigneten Träger gebunden. Dazu kann z.B. Sepharose wie nachstehend beschrieben, aktiviert werden (H.-F.Boeden, W.Büttner, C.Rupprich, D.Büttner, S.Heinrich, M.Becker, M.Holtzhauer (1992) Makromol. Chem. ***193,*** 865-887):
Der Agarose-Träger wird stufenweise, d.h. durch eine um 20 % schrittweise sich erhöhende Menge an Aceton überführt. Zum Schluß wird der Träger in einem fünffachen Bettvolumen mit wasserfreiem Aceton in einem verschlossenen Gefäß über
Nacht stehen gelassen und nochmals mit 5 bis 10 Vol. wasserfreiem Aceton gewaschen und kurz auf einer G2-Fritte abgesaugt. Zu 10 ml sedimentiertem Träger werden 400 mg N-(Chlorcarbonyloxy)-5-norbornen-2,3-dicarboximid (CICOONB) in 10 ml wasserfreiem Aceton p.a. gegeben. Unter Schütteln wird innerhalb von 15 Minuten eine Lösung von 280 µl Triethylamin und 20 mg 4-Dimethylamino-pyridin (DMAP) in 5 ml trockenem Aceton tropfenweise zugegeben (Molverhältnis CICOONB :Triethylamin:DMAP 1:1,2:0,1). Man schüttelt anschließend weitere 15 Minuten und wäscht dann den Träger mit ca. 200 ml wasserfreiem Aceton.

### b) Kopplung der IgY an einen festen Träger

Die nach Beispiel 4a) aktivierte Polysaccarid-Matrix (Gel) wird stufenweise in ein wäßriges Medium überführt und dann sofort in die Kopplungslösung, die den Liganden enthält, eingerührt. Als Kopplungspuffer wird Citratpuffer pH 4,2 verwendet. Die Kopplung erfolgt unter leichtem Schütteln 2 h bei Raumtemperatur. Freie Bindungen werden anschließend durch Zugabe von Ethanolamin blockiert. In der Tab. 2 sind die konkreten Bedingungen für die einzelnen Antikörper dargestellt.

**Tabelle 2**

| Gel Nr. | Chicken-Ak (IgY) | mg | mg/ml | Ak-Lösung (ml) | ml Kopplungspuffer (Citrat, 0,1 M, pH 4,2) | Ethanolamin 1 M (ml) | feuchtes Gel (g) |
|---|---|---|---|---|---|---|---|
| 1 | ChalL1 | 9,5 | 13,5 | 0,7 | 4,3 | 0,5 | 5,55 |
| 2 | ChalL6 | 9,8 | 9,8 | 1,0 | 4,0 | 0,5 | 5,58 |
| 3 | ChalL10 | 9,2 | 7,4 | 1,2 | 3,8 | 0,5 | 5,55 |
| 4 | ChaTNF | 11,0 | 11,6 | 1,0 | 4,1 | 0,5 | 5,56 |
| 5 | ChaLPS | 11,6 | 13,7 | 0,9 | 4,2 | 0,5 | 5,60 |
| 6 | ChaC3a | 6,9 | 10,7 | 0,6 | 4,4 | 0,5 | 5,57 |
| 7 | ChaC5a | 11,3 | 11,1 | 1,0 | 4,0 | 0,5 | 5,55 |
| 8 | Kontrolle | 0,0 | 0,0 | 0,0 | 5,0 | 0,5 | 5,61 |

### Beispiel 5

Die nach Beispiel 4 immobilisierten Antikörper werden benutzt, um aus flüssigen Medien wie Pufferlösungen, Serum oder Blutplasma Lipopolysaccharide, Interleukine, TNF oder Komplementfaktoren zu entfernen.
Dazu werden die Träger gewaschen, in einen physiologischen Puffer (PBS) überführt und luftblasenfrei in Plastik- oder Glassäulen gepackt. Die Anordnung wird durch Anschluß an eine Chromatografieeinrichtung komplettiert. Das zu adsorbierende Probenmaterial (mit den Antigenen dotierter Puffer, Serum- oder Blutplasmaproben, dotiert oder mit natürlichem Antigengehalt) kann nun durch Schwerkraft oder mit einer geeigneten Pumpe über die immobilisierten, für die aufgeführten Antigene spezifischen Antikörper geleitet werden. Die vorhandenen Antigene werden von den IgY erkannt, fest gebunden und somit aus dem die Säule durchströmenden Medium entfernt. Der Nachweis der Wirksamkeit erfolgt durch Analyse (ELISA) des Säulendurchlaufs, dessen Antigengehalt vermindert ist. Nach Waschen der Säule mit physiologischem Puffer erfolgt die Desorption des gebundenen Antigens mit geeigneten Elutionsmitteln (0,1 M Citratpuffer pH 3), Fraktionierung und Analyse des Eluates. Der quantitative Nachweis der Antigene wird zur Kapazitätsbestimmung des Immunosorbents genutzt.

## Patentansprüche

1. Immunadsorber zur Sepsistherapie **gekennzeichnet durch**
Trägermaterialien aus organischen oder synthetischen Polymeren mit gebundenen poly- oder monoklonalen Antikörpern, die gegen die Komplementfaktoren C3a und/oder C5a und gegen Lipopolysaccharide (LPS) sowie ggf. mit Antikörpern, die gegen weitere Sepsis-Mediatoren gerichtet sind.

2. Immunadsorber nach Anspruch 1, **dadurch gekennzeichnet, daß**
die Antikörper polyklonale Antikörper sind.

3. Immunadsorber nach Anspruch 2, **dadurch gekennzeichnet, daß**
die Antikörper aviäre Antikörper des Typs IgY sind.

4. Immunadsorber nach Anspruch 1 bis 3, **dadurch gekennzeichnet, daß**
weitere Antikörper gegen Sepsis-Mediatoren entsprechend des Zustandes der Dysregulation enthalten sind.

5. Immunadsorber nach Anspruch 1 und 4, **dadurch gekennzeichnet, daß**
diese Antikörper gegen TNF, IL1, IL6, IL8 und/oder IL10 gerichtet sind.

6. Immunadsorber nach Anspruch 1, **dadurch gekennzeichnet, daß** die gebundenen Antikörper gegen mindestens eine der folgenden Peptidsequenzen der Komplementfaktoren C3a und C5a 
C3a: NH2-KCCEDGMRQNPMR-COOH
NH2-RFSCQRRTRFISL-COOH
NH2-ITELRRQHARAS-COOH
 
C5a: NH2-QADYKDDDDKLPAE-COOH
NH2-DDKLPAEGLDIENS-COOH
 gerichtet sind.

7. Immunadsorber nach Anspruch 5, **dadurch gekennzeichnet, daß** die gebundenen Antikörper gegen mindestens eine der folgenden Peptidsequenzen der Interleukine 1α und 1β 
IL1α: NH2-NCYSENEEDSSSID-COOH
NH2-GAYKSSKDDAKIT-COOH
NH2-WETHGTKNYFTS-COOH
 
IL1β: NH2-RISDHHYSKGFRQA-COOH
NH2-VQGEESNDKIPVA-COOH
NH2-ESVDPKNYPKKKMEKRF-COOH
 gerichtet sind.

8. Immunadsorber nach Anspruch 5, **dadurch gekennzeichnet, daß** die gebundenen Antikörper gegen mindestens eine der folgenden Peptidsequenzen des Interleukin 6 
IL6:	NH2-APHRQPLTSSERIDKQI- COOH
NH2-QNRFESSEEQARA- COOH
NH2-AITTPDPTTNAS- COOH
 gerichtet sind.

9. Immunadsorber nach Anspruch 5, **dadurch gekennzeichnet, daß** die gebundenen Antikörper gegen mindestens eine der folgenden Peptidsequenzen des Interleukin 10 
IL10: NH2-SPGQGTQSENSCT-COOH
NH2-QMKDQLDNLLLKES-CCOH
NH2-MPQAENQDPDIKA-COOH
NH2-LPCENKSKAVEQ-COOH
 gerichtet sind.

10. Immunadsorber nach Anspruch 5, **dadurch gekennzeichnet, daß** die gebundenen Antikörper gegen mindestens eine der folgenden Peptidsequenzen 
TNFα: NH2-VRSSSRTPSDKPVA-COOH
NH2-KSPCQRETPEGAEAKPW-COOH
 gerichtet sind.

11. Immunadsorber nach den Ansprüchen 1 bis 10, **dadurch gekennzeichnet, daß** das organische oder synthetische Trägermaterial aus Membranen oder Partikeln aus Polystyrolen, Kohlenhydraten, wie Zellulose- oder Agarosederivaten, oder Acrylaten besteht.

12. Immunadsorber nach den Ansprüchen 1 bis 11, **dadurch gekennzeichnet, daß** die spezifischen Antikörper kovalent an die Membranen oder Partikel gebunden sind.

13. Immunadsorber nach den Ansprüchen 1 bis 11, **dadurch gekennzeichnet, daß** die Antikörper über Spacer oder Linker an die Trägermaterialien fixiert sind.

14. Verfahren zur Herstellung von Immunadsorbern gemäß Anspruch 1 bis 13, dadurch gekenzeichnet, daß an Trägermateriajen aus organischen oder synthetischen Polymeren Antikörper, die gegen C3a und/oder C5a und LPS sowie ggf. gegen weitere Sepsis-Mediatoren gerichtet sind, kovalent oder adsorptiv gekoppelt werden.

15. Verfahren nach Anspruch 14, **dadurch gekennzeichnet, daß** die Antikörper durch Immunisierung vorzugsweise von Kleinsäugern, wie Mäusen, Ratten oder Kaninchen, oder Vögeln, wie Hühnern, mit den entsprechenden Antigenen hergestellt werden.

16. Verwendung von Immunadsorbern gemäß Anspruch 1 bis 13 als wirksamer Bestandteil einer Vorrichtung zur Entfernung von Komplementfaktoren, LPS und ggf. weiteren Mediatoren in patientenspezifischer Kombination aus Körperflüssigkeiten.

17. Verwendung nach Anspruch 16, **dadurch gekennzeichnet, daß** die Immunadsorber für die Plasmapherese bei Patienten mit Sepsis oder septischem Schock sowie anderen Krankheiten, die mit Entzündungen einhergehen, geeignet sind.

## Claims

1. Immunoadsorbers for sepsis therapy, wherein
there exist carrier materials of organic or synthetic polymers with bound polyclonal or monoclonal antibodies aimed against the complementary factors C3a and/or C5a and against lipo-polysaccharides (LPS) and, if need be, with antibodies aimed against further sepsis mediators.

2. Immunoadsorbers according to Claim 1, wherein the antibodies are polyclonal antibodies.

3. Immunoadsorbers according to Claim 2, wherein the antibodies are aviary antibodies of type IgY.

4. Immunoadsorbers according to Claims 1 to 3, wherein further antibodies against sepsis mediators in accordance with the state of the dysregulation are contained.

5. Immunoadsorbers according to Claims 1 and 4, wherein these antibodies are aimed against TNF, IL1, IL6, IL8 and/or IL10.

6. Immunoadsorbers according to claim 1, wherein the bound antibodies are aimed against at least one of the following peptide sequences of the complementary factors C3a and C5a:

C3a: NH2-KCCEDGMRQNPMR-COOH
NH2-RFSCQRRTRFISL-COOH
NH2-ITELRRQHARAS-COOH

C5a: NH2-QADYKDDDDKLPAE-COOH
NH2-DDKLPAEGLDIENS-COOH

7. Immunoadsorber according to Claim 5, wherein the bound antibodies are aimed against at least one of the following peptide sequences of the interleukins 1α and 1β:

IL1α: NH2-NCYSENEEDSSSID-COOH
NH2-GAYKSSKDDADKIT-COOH
NH2-WETHGTKNYFTS-COOH

IL1β:   NH2-RISDHHYSKGFRQA-COOH
        NH2-VQGEESNDKIPVA-COOH
        NH2-ESVDPKNYPKKKMEKRF-COOH

8. Immunoadsorber according to Claim 5, wherein the bound antibodies are aimed against at least one of the following peptide sequences of interleukin 6:

IL6: NH2-APHRQPLTSSERIDKQI-COOH
NH2-QNRFESSEEQARA-COOH
NH2-AITTPDPTTNAS-COOH

9. Immunoadsorber according to Claim 5, wherein the bound antibodies are aimed against at least one of the following peptide sequences of interleukin 10:

IL10: NH2-SPGQGTQSENSCT-COOH
NH2-QMKDQLDNLLLKES-COOH
NH2-MPQAENQDPDIKA-COOH
NH2-LPCENKSKAVEQ-COOH

10. Immunoadsorber according to Claim 5, wherein the bound antibodies are aimed against at least one of the following peptide sequences:

TNFα: NH2-VRSSSRTPSDKPVA-COOH
NH2-KSPCQRETPEGAEAKPW-COOH

11. Immunoadsorber according to Claims 1 to 10, wherein the organic or synthetic carrier material comprises membranes or particles of polystyrols, carbohydrates such as cellulose or agarose derivatives or acrylates.

12. Immunoadsorber according to Claims 1 to 11, wherein the specific antibodies are bound covalently to the membranes or particles.

13. Immunoadsorber according to Claims 1 to 11, wherein the antibodies are fixed to the carrier materials by spacers or linkers.

14. Method for the production of Immunoadsorbers according to Claims 1 to 13, wherein antibodies aimed against C3a and/or C5a and LPS and, if applicable, against further sepsis mediators, are covalently or adsorptively coupled onto carrier materials of organic or synthetic polymers.

15. Method according to Claim 14, wherein the antibodies are produced by immunisation, preferably of small mammals such as mice, rats or rabbits, or birds, such as chickens, with the corresponding antigens.

16. Use of Immunoadsorbers according to Claims 1 to 13 as the effective integral part of a device for the removal of complementary factors, LPS and, if applicable, further mediators in a patient-specific combination from body fluids.

17. Use according to Claim 16, wherein the Immunoadsorbers are suitable for plasmapheresis in patients with sepsis or septic shock and other diseases connected with inflammations.

## Revendications

1. Absorbant immunitaire pour le traitement de la septicémie se caractérisant par des matériaux supports composés de polymères organiques ou synthétiques avec des anticorps polyclonaux ou monoclonaux qui sont orientés contre les facteurs complémentaires C3a et/ou C5a et contre les lipopolysacchardies (LPS) ainsi que le cas échéant avec des anticorps qui s'orientent contre d'autres médiateurs de septicémie.

2. Absorbant immunitaire selon la revendication 1, se caractérisant par le fait que les anticorps sont des anticorps polyclonaux.

3. Absorbant immunitaire selon la revendication 2, se caractérisant par le fait que les anticorps sont des anticorps aviaires de type IgY.

4. Absorbant immunitaire selon la revendication 1 à 3, se caractérisant par le fait qu'ils contiennent d'autres anticorps dirigés contre des médiateurs de septicémie selon l'état de dérégulation.

5. Absorbant immunitaire selon la revendication 1 et 4, se caractérisant par le fait que ces anticorps sont des anti-TNF, anti-ILI, IL6, IL8 et/ou IL10.

6. Absorbant immunitaire selon la revendication 1, se caractérisant par le fait que les anticorps liés sont orientés contre au moins une des séquences peptides suivantes des facteurs complémentaires C3a et C5a

C3a: NH2-KCCEDGMRQNPMR-COOH
NH2-RFSCQRRTRFISL-COOH
NH2-ITELRRQHARAS-COOH

C5a: NH2-QADYKDDDDKLPAE-COOH
NH2-DDKLPAEGLDIENS-COOH.

7. Absorbant immunitaire selon la revendication 5, se caractérisant par le fait que les anticorps liés sont orientés contre au moins une des séquences peptides suivantes des interleukines 1α et 1β

IL1α:	NH2-NCYSENEEDSSSID-COOH
NH2-GAYKSSKDDAKIT-COOH
NH2-WETHGTKNYFTS-COOH

IL1β :    NH2-RISDHHYSKGFRQA-COOH
NH2-VQGEESNDKIPVA-COOH
NH2-ESVDPKNYPKKKMEKRF-COOH

8. Absorbant immunitaire selon la revendication 5, se caractérisant par le fait que les anticorps liés sont orientés contre au moins une des séquences peptides suivantes de l'interleukine 6

IL6: NH2-APHRQPLTSSERIDKQI-COOH
NH2-QNRFESSEEQARA-COOH
NH2-AITTPDPTTNAS-COOH.

9. Absorbant immunitaire selon la revendication 5, se caractérisant par le fait que les anticorps liés sont orientés contre au moins une des séquences peptides suivantes de l'interleukine 10

IL10: NH2-SPGQGTQSENSCT-COOH
NH2-QMKDQLDNLLLKES-COOH
NH2-MPQAENQDPDIKA-COOH
NH2-LPCENKSKAVEQ-COOH.

10. Absorbant immunitaire selon la revendication 5, se caractérisant par le fait que les anticorps liés sont orientés contre au moins une des séquences peptides suivantes

TNFα: NH2-VRSSSRTPSDKPVA-COOH
NH2-KSPCQRETPEGAEAKPW-COOH.

11. Absorbant immunitaire selon les revendications 1 à 10, se caractérisant par le fait que le matériau porteur organique ou synthétique se compose de membranes ou de particules de polystyrènes, hydrates de carbones, tels que les dérivés de cellulose ou d'agarose ou d'acrylates.

12. Absorbant immunitaire selon les revendications 1 à 11, se caractérisant par le fait que les anticorps spécifiques sont liés en covalence aux membranes ou particules.

13. Absorbant immunitaire selon les revendications 1 à 11, se caractérisant par le fait que les anticorps sont fixés aux matériaux porteurs par l'intermédiaire d'espaceurs ou de lieurs.

14. Procédé de fabrication d'absorbants immunitaires selon la revendication 1 à 13, se caractérisant par le fait que des anticorps qui sont orientés contre C3a et/ou C5a et LPS ainsi que le cas échéant contre d'autres médiateurs de septicémie, sont couplés en covalence ou absorption aux matériaux porteurs composés de polymères organiques ou synthétiques.

15. Procédé de fabrication d'absorbants immunitaires selon la revendication 14, se caractérisant par le fait que les anticorps sont fabriqués par le biais de l'immunisation préférentielle de petits mammifères, tels que les souris, les rats ou les lapins, ou d'oiseaux, tels que les poules, avec les antigènes appropriés.

16. Emploi d'absorbants immunitaires selon la revendication 1 à 13 en tant qu'élément actif d'un dispositif destiné à éliminer des facteurs complémentaires, des LPS et le cas échéant d'autres médiateurs combinés aux liquides organiques spécifiques du patient.

17. Emploi selon la revendication 16, se caractérisant par le fait que les absorbants immunitaires sont appropriés à la plasmaphérèse chez des patients souffrant de septicémie ou sous choc septicémique ainsi que d'autres maladies qui sont accompagnées d'inflammations.
